# EUROPEAN PATENT SPECIFICATION

(11) **EP 2 706 979 B1**
(45) Date of publication and mention of the grant of the patent: **04.07.2018**
(21) Application number: 12719694.7
(22) Date of filing: 04.05.2012
(51) Int. Cl.: A61K 8/898, A61Q 5/12

(54) **HAIR TREATMENT COMPOSITIONS**
HAARBEHANDLUNGSZUSAMMENSETZUNGEN
COMPOSITIONS DE TRAITEMENT CAPILLAIRE

(30) Priority: 12.05.2011 EP 11165889
(43) Date of publication of application: 19.03.2014
(73) Proprietor: Unilever PLC, London, Greater London EC4Y 0DY (GB); Unilever N.V., 3013 AL Rotterdam (NL)
(72) Inventor: DERICI, Leo, Wirral, Merseyside CH63 3JW (GB); MURRAY, Andrew, Malcolm, Wirral, Merseyside CH63 3JW (GB); RICHARDS, Claire, Louise, Wirral, Merseyside CH63 3JW (GB)
(74) Representative: Chisem, Janet
(86) International application number: PCT/EP2012/058306
(87) International publication number: WO 2012/152723

(56) References cited:
- EP-A2- 0 455 185
- WO-A1-99/29286
- WO-A2-02/096375
- JP-A- 8 217 644

## Description

### HAIR TREATMENT COMPOSITION

The current invention relates to the use of a hair treatment composition to provide selective conditioning benefits to damaged hair.

### BACKGROUND AND PRIOR ART

WO9929286 discloses an aqueous hair treatment composition comprising an amino functionalised silicone and emulsified particles of insoluble, hydroxy functionalised silicone. Suitable amino functionalised silicones are DC2-8220 and DC2-8466. The composition is selective to target the areas of the hair which need it most, such as the damaged ends. The compositions are preferably shampoos or conditioners. The conditioning compositions will comprise conditioning surfactants, particularly cationic conditioning surfactants. The amine numbers of DC2-8220 and DC2-8466 are 1.25 and 1.36 respectively. WO02/096375 (Unilever) discloses a blend of silicones comprising a combination of a first silicone having a viscosity of at least 100,000 m²/sec and a second amino functionalised silicone provides excellent conditioning benefits.

WO99/44565 and WO99/44567 (Unilever) disclose shampoo compositions containing a combination of an amino-functionalised silicone and an insoluble non-amino functional insoluble silicone.

WO99/49836 (Unilever) discloses rinse-off conditioner formulations containing an amino-functional silicone corresponding to a defined general formula and having a mole percent amino functionality of at least 1 mole %. The formulations may further comprise emulsified particles of a non-amino functionalised silicone.

We have surprisingly found that amino-silicones can selectively deposit silicone mixtures onto the hair such that their deposition is enhanced on areas where the hair is damaged.

### SUMMARY OF THE INVENTION

The present invention relates to the use of an amino-functionalized silicone having an amine number from 0.1 to 0.6 within a conditioning formulation to selectively enhance silicone deposition onto damaged hair; in which the conditioning composition further comprises a cationic surfactant.

### DETAILED DESCRIPTION OF THE INVENTION

Unless specified otherwise, all wt% values quoted hereinafter are percentages by weight based on total weight of the hair treatment composition.

The amine number is the weight in milligrams of KOH equivalent to the total amine hydrogen content in one gram of amino functionalized silicone. The amine number is determined by titration of the amine acetate ion by a dilute, typically 1N HCl solution. For pure material, the amine number can be calculated using the molecular weights of the pure compound and KOH (56.1 g/mol).

Unless stated otherwise the viscosity of silicones can be measured by means of a glass capillary viscometer as set out further in Dow Corning Corporate Test Method CTM004, July 20 1970.

Silicone particle size may be measured by means of a laser light scattering technique, for example using a 2600D Particle Sizer from Malvern Instruments.

The term amino-silicone and amino-functionalised silicone are used interchangeably within this specification.

Damaged hair is hair fibre damage that has occurred by mechanical or chemical means or a combination of both. It can include cuticle loss and/or erosion, longitudinal rupturing, and fibril disintegration.

### Silicone Component

The total silicone content of the composition of the invention is suitably in the region of from 0.05 to 20%, preferably from 1 to 10 wt%.

Preferably, the D_{3,2} average particle size of the silicone droplets in the emulsion and also in the final composition is less than 100 µm, more preferably less than 20 µm, and yet more preferably less than 10 µm. Preferably, the average particle size of the silicone droplets in the emulsion and also in the final composition is greater than 0.1 µm. A smaller silicone particle size enables a more uniform distribution of silicone on the hair for the same amount of silicone in the composition.

### Amine Functionalised Silicone

The composition of the invention disclose the use of an amino functionalised silicone.

The amino functionalised silicone emulsion preferably has a viscosity of less than 500,000 mm2/sec at 25°C, more preferably less than 400,000 mm2/sec at 25°C, most preferably less than 200,000 mm2/sec at 25°C.

Suitably, the amino functionalised silicone has a molecular weight less than 200,000 Dalton, preferably less than 100,000 Dalton, more preferably in the range from 1 to 80,000 Dalton.

Suitable amino functionalised silicones are described in EP 455,185 (Helene Curtis) and include trimethylsilylamodimethicone as depicted below, and are sufficiently water insoluble so as to be useful in compositions of the invention: Si(CH₃)₃-O-[Si(CH₃)₂-O-]ₓ-[Si(CH₃)(R-NH-CH₂CH₂NH₂)-O-]_{y}-Si (CH₃)₃
wherein x + y is a number from about 50 to about 500, and the mole % amine functionality is in the range of from about 0.3 to about 8%, and wherein R is an alkylene group having from 2 to 5 carbon atoms. Preferably, the number x + y is in the range of from about 100 to about 300, and the mole % amine functionality is in the range of from about 1.5 to about 6%.

Examples of amino-functionalised silicones useful in the silicone component of the composition of the invention include ADM22 ex Wacker.

The amino-silicone has an amine number from 0.1 to 0.6, more preferably from 0.2 to 0.5, most preferably from 0.30 to 0.45.

Preferably the amino-silicone is present at a level from 0.05 to 10 wt% of the total composition, more preferably form 0.1 to 5 wt%.

Preferably the amino-silicone is emulsified.

In one preferred embodiment the amino-silicone is emulsified with a non-functionalised silicone. It is preferred if the amino-silicone is present with a non-functional silicone as a silicone blend emulsion.

In the context of the present invention a silicone blend emulsion is defined as a mixture of silicones that are mixed prior to emulsification. This product emulsion type means that each silicone droplet in the emulsion will have essentially the same composition and will comprise a mixture (typically a solution) of the two types of silicone which together make up the silicone component of the composition, i.e. first silicone and second silicone.

Preferably the silicone blend emulsion is an aqueous emulsion, more preferably the aqueous emulsion is mechanically-formed. In such emulsions, it is preferable that the emulsion additionally includes at least one emulsifier in order to stabilise the silicone emulsion.

Suitable emulsifiers are well known in the art and include anionic and nonionic surfactants. Examples of anionic surfactants used as emulsifiers for the silicone particles are alkylarylsulphonates, e.g., sodium dodecylbenzene sulphonate, alkyl sulphates e.g., sodium lauryl sulphate, alkyl ether sulphates, e.g., sodium lauryl ether sulphate nEO, where n is from 1 to 20 alkylphenol ether sulphates, e.g., octylphenol ether sulphate nEO where n is from 1 to 20, and sulphosuccinates, e.g., sodium dioctylsulphosuccinate.

Examples of nonionic surfactants used as emulsifiers for the silicone particles are alkylphenol ethoxylates, e.g., nonylphenol ethoxylate nEO, where n is from 1 to 50, alcohol ethoxylates, e.g., lauryl alcohol nEO, where n is from 1 to 50, ester ethoxylates, e.g., polyoxyethylene monostearate where the number of oxyethylene units is from 1 to 30.

It is preferable if the non-functionalised silicone of the emulsion has a viscosity of at least 100,000 mm2/sec at 25°C, preferably at least 200,000 mm2/sec at 25°C, more preferably at least 400,000 mm2/sec at 25°C.

In a preferred embodiment, the non-functionalised silicone is a silicone gum and has a viscosity of at least 500,000 mm2/sec at 25°C, more preferably at least 600,000 mm²/sec at 25°C, and yet more preferably at least 1,000,000 mm²/sec at 25°C.

Suitably, the non-functionalised silicone has a molecular weight of at least 100,000 Dalton, and preferably at least 200,000 Dalton. When the first silicone is gum, the molecular weight is suitably at least 400,000 Dalton, preferably at least 500,000 Dalton, and more preferably at least 550,000 Dalton.

Suitable as the non-functionalised silicone is a polydiorganosiloxane, preferably derived from suitable combinations of R₃SiO_{0.5} and R₂SiO units, where each R independently represents an alkyl, alkenyl (e.g. vinyl), alkaryl, aralkyl or aryl (e.g. phenyl) group. R is most preferably methyl. Thus, preferred first silicones for use in the silicone component of compositions of the invention are polydimethylsiloxanes (which have the CTFA designation dimethicone), optionally having end groups such as hydroxyl. Good results have been obtained with dimethicone.

Suitably, the weight ratio of the non-functionalised silicone to aminosilicone is in the range from 15:1 to 1:1, preferably from 10:1 to 1:1, more preferably from 8:1 to 1:1, and yet more preferably from 6:1 to 2:1. A particularly preferred ratio is 3:1.

WO02/096375 (Unilever) describes examples of suitable silicone blend emulsions and their method of manufacture.

### Conditioning Surfactant

Conditioner according to the invention will comprise one or more cationic conditioning surfactants which are cosmetically acceptable and suitable for topical application to the hair, used singly or in admixture.

Cationic surfactants useful in compositions of the invention contain amino or quaternary ammonium hydrophilic moieties which are positively charged when dissolved in the aqueous composition of the present invention.

Examples of suitable cationic surfactants are those corresponding to the general formula:

[N(R₁)(R₂)(R₃)(R₄)]⁺ (X)⁻

in which R₁, R₂, R₃, and R₄ are independently selected from (a) an aliphatic group of from 1 to 22 carbon atoms, or (b) an aromatic, alkoxy, polyoxyalkylene, alkylamido, hydroxyalkyl, aryl or alkylaryl group having up to 22 carbon atoms; and X is a salt-forming anion such as those selected from halogen, (e.g. chloride, bromide), acetate, citrate, lactate, glycolate, phosphate nitrate, sulphate, and alkylsulphate radicals.

The aliphatic groups can contain, in addition to carbon and hydrogen atoms, ether linkages, and other groups such as amino groups. The longer chain aliphatic groups, e.g., those of about 12 carbons, or higher, can be saturated or unsaturated.

The most preferred cationic surfactants for conditioner compositions of the present invention are monoalkyl quaternary ammonium compounds in which the alkyl chain length is C8 to C14.
Suitable examples of such materials correspond to the general formula:

[N(P₅)(P₆)(P₇)(P₈)]⁺ (X)⁻

in which R₅ is a hydrocarbyl chain having 8 to 14 carbon atoms or a functionalised hydrocarbyl chain with 8 to 14 carbon atoms and containing ether, ester, amido or amino moieties present as substituents or as linkages in the radical chain, and R₆, R₇ and R₈ are independently selected from (a) hydrocarbyl chains of from 1 to about 4 carbon atoms, or (b) functionalised hydrocarbyl chains having from 1 to about 4 carbon atoms and containing one or more aromatic, ether, ester, amido or amino moieties present as substituents or as linkages in the radical chain, and X is a salt-forming anion such as those selected from halogen, (e.g. chloride, bromide), acetate, citrate, lactate, glycolate, phosphate nitrate, sulphate, and alkylsulphate radicals.

The functionalised hydrocarbyl chains (b) may suitably contain one or more hydrophilic moieties selected from alkoxy (preferably C₁ - C₃ alkoxy), polyoxyalkylene (preferably C₁ - C₃ polyoxyalkylene), alkylamido, hydroxyalkyl, alkylester, and combinations thereof.

Preferably the hydrocarbyl chains R₁ have 12 to 14 carbon atoms, most preferably 12 carbon atoms. They may be derived from source oils which contain substantial amounts of fatty acids having the desired hydrocarbyl chain length. For example, the fatty acids from palm kernel oil or coconut oil can be used as a source of C8 to C12 hydrocarbyl chains.

Typical monoalkyl quaternary ammonium compounds of the above general formula for use in shampoo compositions of the invention include:
(i) lauryl trimethylammonium chloride(available commercially as Arquad C35 ex-Akzo); cocodimethyl benzyl ammonium chloride (available commercially as Arquad DMCB-80 ex-Akzo)
(ii) compounds of the general formula:

   [N(R₁)(R₂)((CH₂CH₂O)ₓH)((CH₂CH₂O)_{y}H)]⁺ (X)⁻

   in which:
   x + y is an integer from 2 to 20;
   R₁ is a hydrocarbyl chain having 8 to 14, preferably 12 to 14, most preferably 12 carbon atoms or a functionalised hydrocarbyl chain with 8 to 14, preferably 12 to 14, most preferably 12 carbon atoms and containing ether, ester, amido or amino moieties present as substituents or as linkages in the radical chain;
   R₂ is a C₁ - C₃ alkyl group or benzyl group, preferably methyl, and
   X is a salt-forming anion such as those selected from halogen, (e.g. chloride, bromide), acetate, citrate, lactate, glycolate, phosphate nitrate, sulphate, methosulphate and alkylsulphate radicals.

Suitable examples are PEG-n lauryl ammonium chlorides (where n is the PEG chain length), such as PEG-2 cocomonium chloride (available commercially as Ethoquad C12 ex-Akzo Nobel); PEG-2 cocobenzyl ammonium chloride (available commercially as Ethoquad CB/12 ex-Akzo Nobel); PEG-5 cocomonium methosulphate (available commercially as Rewoquat CPEM ex-Rewo); PEG-15 cocomonium chloride (available commercially as Ethoquad C/25 ex-Akzo).
(iii) compounds of the general formula:

[N(R₁)(R₂)(R₃)((CH₂)ₙOH)]⁺ (X)⁻

in which:
n is an integer from 1 to 4, preferably 2;
R₁ is a hydrocarbyl chain having 8 to 14, preferably 12 to 14, most preferably 12 carbon atoms;
R₂ and R₃ are independently selected from C₁ - C₃ alkyl groups, and are preferably methyl, and
X is a salt-forming anion such as those selected from halogen, (e.g. chloride, bromide), acetate, citrate, lactate, glycolate, phosphate nitrate, sulphate, and alkylsulphate radicals.

Suitable examples are lauryldimethylhydroxyethylammonium chloride (available commercially as Prapagen HY ex-Clariant).

Mixtures of any of the foregoing cationic surfactants compounds may also be suitable.

Examples of suitable cationic surfactants include:
quaternary ammonium chlorides, e.g. alkyltrimethylammonium chlorides wherein the alkyl group has from about 8 to 22 carbon atoms, for example octyltrimethylammonium chloride, dodecyltrimethylammonium chloride, hexadecyltrimethyl-ammonium chloride, cetyltrimethylammonium chloride, octyldimethylbenzylammonium chloride, decyldimethylbenzyl-ammonium chloride, stearyldi-methylbenzylammonium chloride, didodecyldimethylammonium chloride, dioctadecyldimethylammonium chloride, tallow trimethylammonium chloride, cocotrimethylammonium chloride, and the corresponding salts thereof, e.g., bromides, hydroxides. Cetylpyridinium chloride or salts thereof, e.g., chloride Quaternium -5
Quaternium -31
Quaternium -18
and mixtures thereof.

In the conditioners of the invention, the level of cationic surfactant is preferably from 0.01 to 10, more preferably 0.05 to 5, most preferably 0.1 to 2 wt% of the total composition.

### Fatty Alcohol

Conditioners of the invention advantageously incorporate a fatty alcohol material. The combined use of fatty alcohol materials and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 20. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol material in conditioners of the invention is conveniently from 0.01 to 10, preferably from 0.1 to 5 wt% by weight of the total composition. The weight ratio of cationic surfactant to fatty alcohol is suitably from 10:1 to 1:10, preferably from 4:1 to 1:8, optimally from 1:1 to 1:4.

### Mousses

Hair treatment compositions in accordance with the invention may also take the form of aerosol foams (mousses) in which case a propellant must be included in the composition. This agent is responsible for expelling the other materials from the container and forming the hair mousse character.

The propellant gas can be any liquefiable gas conventionally used for aerosol containers. Examples of suitable propellants include dimethyl ether, propane, n-butane and isobutane, used singly or in admixture.

The amount of the propellant gases is governed by normal factors well known in the aerosol art. For hair mousses, the level of propellant is generally from 3 to 30, preferably from 5 to 15 wt% of the total composition.

Small quantities of surfactant ranging anywhere from 0.1 to 10, preferably from 0.1 to about 1 wt%, for example 0.3 wt% may be present in the hair mousse compositions of the invention. The surfactant may be an anionic, nonionic or cationic emulsifier. Particularly preferred are nonionic emulsifiers which are formed from alkoxylation of hydrophobes such as fatty alcohols, fatty acids and phenols.

### Optional Ingredients

Compositions of this invention may contain any other ingredient normally used in hair treatment formulations. These other ingredients may include viscosity modifiers, preservatives, colouring agents, polyols such as glycerine and polypropylene glycol, chelating agents such as EDTA, antioxidants, fragrances, antimicrobials and sunscreens. Each of these ingredients will be present in an amount effective to accomplish its purpose. Generally these optional ingredients are included individually at a level of up to 5 wt% of the total composition. Preferably, compositions of this invention also contain adjuvants suitable for hair care. Generally such ingredients are included individually at a level of up to 2, preferably up to 1 wt% of the total composition.

Among suitable hair care adjuvants, are:
(i) natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts. A particularly preferred combination of natural hair root nutrients for inclusion in compositions of the invention is isoleucine and glucose. A particularly preferred amino acid nutrient is arginine.
(ii) hair fibre benefit agents. Examples are:
   - ceramides, for moisturising the fibre and maintaining cuticle integrity. Ceramides are available by extraction from natural sources, or as synthetic ceramides and pseudoceramides. A preferred ceramide is Ceramide II, ex Quest. Mixtures of ceramides may also be suitable, such as Ceramides LS, ex Laboratoires Serobiologiques.

### Mode of Use

The compositions of the invention are primarily intended for topical application to the hair and/or scalp of a human subject to improve hair fibre surface properties such as smoothness, softness, manageability, cuticle integrity, and shine.
The invention will now be further illustrated by the following, non-limiting Examples:

### EXAMPLES

The following composition was prepared

**Table 1**

| Chemical name | Tradename | % of Material in the formulation |
|---|---|---|
| Lactic Acid | Purac HS88 | 0.38 |
| Methyl-p-hydroxy benzoate | Nipagin M | 0.20 |
| Stearyl Alcohol | Lanette S3 | 5.00 |
| Behentrimonium Chloride | Genamin BTLF | 1.24 |
| Stearylamidopropyl dimethylamine | Lexamine S13 | 1.25 |
| Silicone | Examples A, B and 1 | 2.50 |
| Water | Water | To 100 |

**Table 2**

| Example | Silicone | Amine number | Relative Selectivity (%) |
|---|---|---|---|
| A | DC-7134* | 0.63* | 100 |
| B | DC-949 | 0.083 | 103 |
| 1 | ADM22 | 0.32 | 320 |

| | | | |
|---|---|---|---|
| * Amine number of the amino-functionalised silicone component of the blend where the blend comprises a high viscosity dimethicone fluid with a low viscosity amino-functionalised silicone. | | | |

ADM22 is an amino functionalised silicone fluid at the amine number indicated.

The selectivity is the ratio of silicon ppm on the 1 times bleached hair samples to silicon ppm on virgin hair samples.

The relative selectivity, expressed as a percentage, is obtained by ratio-ing the selectivity for each example to the selectivity for example A.

The relative deposition, expressed as a percentage is obtained by ratio-ing the deposition for each example to the deposition for example A on bleached hair.

## Claims

1. Use of an amino-functionalized silicone having an amine number from 0.1 to 0.6 within a conditioning formulation to selectively enhance silicone deposition onto damaged hair; in which the conditioning composition further comprises a cationic surfactant.

2. Use according to claim 1 in which the amine number of the amino-functionalised silicone is from 0.2 to 0.5.

3. Use according to any preceding claim, in which the amino-silicone has a molecular weight of less than 200,000 Daltons.

4. Use according to any preceding claim in which the amino-silicone has an average D_{3,2} particle size in the range from 0.01 to 100 µm, measured by means of a laser light scattering technique.

5. Use according to any preceding claim in which the amino-silicone is emulsified and said emulsion has a viscosity of less than 500,000mm2/sec at 25°C, measured by means of a glass capillary viscometer in Dow Corning Corporate Test Method CTM004.

6. Use according to claim 5 in which the amino-silicone emulsion further comprises a non-functionalised silicone.

7. Use according to any claim 6 in which the weight ratio of the non-functionalised silicone to the amino-silicone in the emulsion is in the range from 15:1 to 1:1.

8. Use according to claim 6 or claim 7 in which the amino-silicone and non-functionalised silicone are mixed prior to emulsification.

9. Use according to any one of claims 6 to 8 in which the amino-silicone and non-functionalised silicone are present within silicone droplets within the amino-silicone and non-functionalised silicone emulsion and are uniformly distributed and in intimate proximity with each other within the silicone droplet.

## Patentansprüche

1. Verwendung eines Amino-funktionalisierten Silicons mit einer Aminzahl von 0,1 bis 0,6 in einer Konditionierungsformulierung, um die Ablagerung von Silicon auf geschädigtem Haar selektiv zu verstärken, wobei die Konditionierungszusammensetzung ferner ein kationisches Tensid aufweist.

2. Verwendung nach Anspruch 1,
wobei die Aminzahl des Amino-funktionalisierten Silicons 0,2 bis 0,5 beträgt.

3. Verwendung nach einem vorstehenden Anspruch,
wobei das Aminosilicon ein Molekulargewicht von weniger als 200000 Dalton aufweist.

4. Verwendung nach einem vorstehenden Anspruch,
wobei das Aminosilicon eine mittlere Teilchengröße D_{3,2} in dem Bereich von 0,01 bis 100 µm, gemessen mittels einer Laserlichtstreuungstechnik, aufweist.

5. Verwendung nach einem vorhergehenden Anspruch, wobei das Aminosilicon emulgiert wird und die Emulsion eine Viskosität von weniger als 500000 mm²/s bei 25°C, gemessen mittels eines Glaskapillarviskosimeters nach dem Dow Corning Corporate-Testverfahren CTM 004, aufweist.

6. Verwendung nach Anspruch 5,
wobei die Aminosilicon-Emulsion ferner ein nicht-funktionalisiertes Silicon aufweist.

7. Verwendung nach Anspruch 6,
wobei das Gewichtsverhältnis des nicht-funktionalisierten Silicons zu dem Aminosilicon in der Emulsion in dem Bereich von 15:1 bis 1:1 liegt.

8. Verwendung nach Anspruch 6 oder Anspruch 7,
wobei das Aminosilicon und das nicht-funktionalisierte Silicon vor dem Emulgieren gemischt werden.

9. Verwendung nach irgendeinem der Ansprüche 6 bis 8,
wobei das Aminosilicon und das nicht-funktionalisierte Silicon in der Emulsion von Aminosilicon und nicht-funktionalisiertem Silicon innerhalb von Silicontröpfchen vorliegen und innerhalb der Silicontröpfchen gleichmäßig verteilt und nahe beieinander vorliegen.

## Revendications

1. Utilisation d'un silicone amino-fonctionnalisé présentant un nombre amine de 0,1 à 0,6 dans une formulation de conditionnement pour promouvoir de manière sélective un dépôt de silicone sur des cheveux abimés ; dans laquelle la composition de conditionnement comprend de plus un tensioactif cationique.

2. Utilisation selon la revendication 1 dans laquelle le nombre amine du silicone amino-fonctionnalisé est de 0,2 à 0,5.

3. Utilisation selon l'une quelconque des revendications précédentes dans laquelle l'amino-silicone présente une masse moléculaire inférieure à 200 000 Daltons.

4. Utilisation selon l'une quelconque des revendications précédentes dans laquelle l'amino-silicone présente une taille moyenne de particule D_{3,2} dans l'intervalle de 0,01 à 100 µm, mesurée au moyen d'une technique de dispersion de lumière laser.

5. Utilisation selon l'une quelconque des revendications précédentes dans laquelle l'amino-silicone est émulsionné et ladite émulsion présente une viscosité inférieure à 500 000 mm²/s à 25°C, mesurée au moyen d'un viscosimètre à capillaire de verre dans la méthode de test Dow Corning Corporate CTM004.

6. Utilisation selon la revendication 5 dans laquelle l'émulsion d'amino-silicone comprend de plus un silicone non-fonctionnalisé.

7. Utilisation selon la revendication 6 dans laquelle le rapport massique du silicone non-fonctionnalisé à l'amino-silicone dans l'émulsion se trouve dans l'intervalle de 15:1 à 1:1.

8. Utilisation selon la revendication 6 ou la revendication 7 dans laquelle l'amino-silicone et le silicone non-fonctionnalisé sont mélangés avant l'émulsification.

9. Utilisation selon l'une quelconque des revendications 6 à 8 dans laquelle l'amino-silicone et le silicone non-fonctionnalisé sont présents dans des gouttelettes de silicone dans l'amino-silicone et l'émulsion de silicone non-fonctionnalisé et sont uniformément distribués et dans une proximité intime l'un avec l'autre dans la gouttelette de silicone.
